Europäisches Patentamt

European Patent Office

Office européen des brevets

(19)

(11) Veröffentlichungsnummer : **0 096 798**
**B1**

(12) **EUROPÄISCHE PATENTSCHRIFT**

(45) Veröffentlichungstag der Patentschrift :
30.01.85

(21) Anmeldenummer : 83105359.0

(22) Anmeldetag : 31.05.83

(51) Int. Cl.⁴ : **C 07 C 27/00, C 07 C 27/12,
C 07 C 35/08, C 07 C 49/403,
B 01 J 29/04, B 01 J 29/14**

(54) **Verfahren zur Herstellung von Cyclohexanol und Cyclohexanon.**

(30) Priorität : 11.06.82 DE 3222144

(43) Veröffentlichungstag der Anmeldung :
28.12.83 Patentblatt 83/52

(45) Bekanntmachung des Hinweises auf die Patenterteilung : 30.01.85 Patentblatt 85/05

(84) Benannte Vertragsstaaten :
**BE CH DE FR GB IT LI NL**

(56) Entgegenhaltungen :
**AT-B- 280 985
DE-A- 2 352 378
DE-B- 1 002 754
US-A- 2 851 496**

(73) Patentinhaber : **BASF Aktiengesellschaft
Carl-Bosch-Strasse 38
D-6700 Ludwigshafen (DE)**

(72) Erfinder : **Hartig, Juergen, Dr.
In der Schleit 9
D-6718 Gruenstadt (DE)**
Erfinder : **Stoessel, Armin, Dr.
Immengaertenweg 20
D-6710 Frankenthal (DE)**
Erfinder : **Herrmann, Guenter, Dr.
Haeuserstrasse 21
D-6900 Heidelberg (DE)**
Erfinder : **Marosi, Laszlo, Dr.
Leuschnerstrasse 32
D-6700 Ludwigshafen (DE)**

**Beschreibung**

Gegenstand der Erfindung ist ein Verfahren zur Herstellung von Cyclohexanol und Cyclohexanon durch Behandeln von Cyclohexylhydroperoxid mit Trägerkatalysatoren, die 2 bis 30 Gew.% Cobalt in oxidischer Form, berechnet als Cobalt, enthalten, bei einer Temperatur von 30 bis 160 °C.

Bei der Oxidation von Cyclohexan mit Luft oder Sauerstoff unter erhöhtem Druck und erhöhter Temperatur erhält man Gemische aus Cyclohexanol, Cyclohexanon, Cyclohexylhydroperoxid, anderen Peroxiden, Säuren und Estern. Zur Verbesserung der Ausbeute wird üblicherweise das gebildete Cyclohexylhydroperoxid unter besonderen Bedingungen zu Gemischen aus Cyclohexanol und Cyclohexanon umgesetzt. In vielen Fällen wird durch Zugabe von speziellen Übergangsmetallsalzen während der Oxidation die gleichzeitige Zersetzung der gebildeten Cyclohexylhydroperoxids bewerkstelligt. So wird in der DE-PS 1 002 754 die Oxidation und die Deperoxidation mit homogen gelösten Cobalt- oder Chromsalzen beschrieben. Auch Kupfer- und Mangansalze erfüllen diese Aufgabe, wie aus der DE-AS 1 193 501 zu entnehmen ist. Diese Verfahren sind jedoch hinsichtlich der Ausbeute verbesserungsbedürftig. Darüber hinaus ist es erforderlich, die Metallsalze wieder zurückzugewinnen.

Es wurden auch schon Trägerkatalysatoren für die Zersetzung von Cyclohexylhydroperoxid enthaltenden Gemischen verwendet. In der US-PS 2 851 496 werden Metalle der 8. Gruppe, z. B. Cobalt auf aktiviertem Aluminiumoxid, Silicagel, Kohle oder Kieselgur, als geeignete Katalysatoren beschrieben. Diese Katalysatoren sind jedoch hinsichtlich ihrer Lebensdauer, Wasser- und Säureempfindlichkeit verbesserungsbedürftig.

Es war deshalb die technische Aufgabe gestellt, für die Zersetzung von Cyclohexylhydroperoxid zu Cyclohexanon und Cyclohexanol Katalysatoren zur Verfügung zu stellen, die eine hohe Ausbeute und hohen Umsatz ermöglichen und zudem eine lange Lebensdauer haben, sowie wenig empfindlich gegenüber Wasser und Säure sind.

Diese Aufgabe wird gelöst in einem Verfahren zur Herstellung von Cyclohexanol und Cyclohexanon durch Behandeln von Cyclohexylhydroperoxid mit Trägerkatalysatoren, die 2 bis 30 Gew.% Cobalt in oxidischer Form, berechnet als Cobalt, enthalten, bei einer Temperatur von 30 bis 160 °C, wobei man Zeolithe als Träger verwendet.

Das neue Verfahren hat den Vorteil, daß die Zersetzung von Cyclohexylhydroperoxid zu Cyclohexanol und Cyclohexanon mit hohen Umsätzen und hohen Ausbeuten verläuft. Zudem hat das neue Verfahren den Vorteil, daß die Katalysatoren eine lange Lebensdauer haben. Ferner hat das neue Verfahren den Vorteil, daß die Katalysatoren wenig empfindlich gegenüber Wasser und Säuren sind, und das aktive Katalysatormetall in geringerem Ausmaß eluiert wird.

Vorteilhaft geht man von Lösungen von Cyclohexylhydroperoxid in Cyclohexan aus. Geeignete Lösungen enthalten beispielsweise 0,5 bis 10 Gew.% Cyclohexylhydroperoxid. Besonders bevorzugt geht man von Gemischen aus, die durch Oxidation von Cyclohexan mit molekularem Sauerstoff oder solchen enthaltenden Gasen, z. B. Luft, in flüssiger Phase bei Temperaturen von 130 bis 200 °C und unter Drücken von 5 bis 25 bar, gegebenenfalls unter Mitverwendung von Katalysatoren wie Cobaltsalzen erhalten wurden. Vorteilhaft werden die so erhaltenen Reaktionsgemische vor der Weiterbehandlung mit Wasser oder Alkalilaugen gewaschen.

Typische Reaktionsgemische enthalten neben Cyclohexan 3 bis 7 Gew.% Cyclohexanon und Cyclohexanol sowie 0,5 bis 3,5 Gew.% Cyclohexylhydroperoxid, ferner Nebenprodukte wie Ester, Carbonsäuren sowie gegebenenfalls Wasser, z. B. bis zu 2 Gew.%. Geeignete Reaktionsgemische erhält man beispielsweise nach dem in der DE-PS 1 046 610 beschriebenen Verfahren.

Die Behandlung erfolgt in Gegenwart von 2 bis 30 Gew.%, insbesondere 10 bis 25 Gew.% Cobalt in oxidischer Form, berechnet als Cobalt, enthaltenden Trägerkatalysatoren, wobei man Zeolithe als Träger verwendet. Die Prozentangaben beziehen sich auf die gesamte Masse aus Träger und dem katalytisch aktiven Metall. Vorteilhaft verwendet man A-, X- oder Y-Zeolithe als Träger. Es hat sich auch bewährt, daß die Katalysatoren einen Gehalt von 3 bis 16 Gew.% Natrium in gebundener Form, berechnet als Natrium, enthalten. Um bei der Katalysatorherstellung eine bessere Verformbarkeit zu erzielen, setzt man vorteilhaft vor der Verformung der Katalysatormasse z. B. bis zu 35 Gew.% aktives Aluminiumoxid- oder -hydroxid, z. B. Böhmit, als Bindemittel zu. In diesem Fall beziehen sich die obigen Prozentangaben auf die gesamte Masse aus dem katalytisch aktiven Metall, Zeolith und Bindemitteln. In besonders bevorzugten Katalysatoren ist das katalytisch aktive Metall in Zeolith eingebaut.

Geeignete Katalysatoren erhält man beispielsweise, indem man auf die genannten Zeolithe Cobaltverbindungen, die beim Erhitzen in ihre Oxide übergehen, aus wäßriger Lösung fällt. Geeignete Cobaltsalze sind beispielsweise Cobaltnitrat, Cobaltsulfat oder Cobaltacetat. Es hat sich als vorteilhaft erwiesen, beim Fällen einen pH-Wert von 7,5 bis 12, insbesondere von 8 bis 11, durch Zugabe einer wäßrigen Alkalihydroxid- oder Carbonatlösung einzuhalten.

Vorteilhaft führt man die Fällung bei einer Temperatur von 40 bis 100 °C durch. Der so erhaltene Cobalthydroxid oder -carbonat enthaltende Zeolithträger wird anschließend mit Wasser ausgewaschen. Vorzugsweise wäscht man mit alkalisiertem Wasser, z. B. bei pH 8 bis 10, aus. Der so erhaltene Trägerkatalysator wird dann vorteilhaft bei 100 bis 150 °C, z. B. für eine Zeit von 2 bis 20 Stunden, getrocknet und anschließend bei einer Temperatur von 200 bis 600 °C für einen Zeitraum von z. B. 2 bis 8 Stunden gesintert. Das Sintern erfolgt zweckmäßig in Gegenwart von molekularem Sauerstoff oder solchen enthaltenden Gasen, z. B. Luft.

Neben der oben ausgeführten bevorzugten Herstellungsweise der verwendeten Katalysatoren ist es auch möglich, Cobaltsalze durch Tränken oder Sprühen aufzubringen und anschließend durch Erhitzen die fertigen Katalysatoren herzustellen.

Sollen die Katalysatoren zu Preßlingen verarbeitet werden, so hat es sich bewährt, vor oder nach dem Sintern, z. B. bis zu 35 Gew.% Böhmit zuzugeben und das so erhaltene Gemisch zu Preßlingen zu verarbeiten. Es wird angenommen, daß Cobalt in Form seiner Oxide vorliegt, obwohl ein röntgenographischer Nachweis dafür nicht immer möglich ist. Es ist auch ein teilweiser Ionenaustausch mit den Zeolithen während der Herstellung nicht auszuschließen.

Die Behandlung des Cyclohexylhydroperoxids oder solches enthaltenden Lösungen erfolgt bei einer Temperatur von 30 bis 160 °C. Besonders gute Ergebnisse erhält man bei einer Temperatur von 80 bis 120 °C. Die Behandlung kann bei Atmosphärendruck oder schwach erhöhtem Druck, z. B. wie er bei der Oxidation von Cyclohexan angewandt wird, z. B. bis zu 20 bar, erfolgen.

Die Behandlung kann absatzweise erfolgen. Vorteilhaft behandelt man in der Technik jedoch Cyclohexylhydroperoxid enthaltende Lösungen und Reaktionsgemische kontinuierlich, indem man solche Lösungen oder Gemische über ein fest angeordnetes Katalysatorbett leitet. Falls nach längerer Betriebszeit die Aktivität der Katalysatoren nachläßt, so lassen sie sich leicht durch Behandeln mit molekularen Sauerstoff enthaltenden Gasen, z. B. Luft, bei einer Temperatur von 150 bis 500 °C reaktivieren.

Das nach dem Verfahren der Erfindung erhältliche Cyclohexanol und Cyclohexanon eignet sich zur Herstellung von Adipinsäure oder Cyclohexanonomin, einem Vorläufer für Caprolactam.

Das Verfahren nach der Erfindung sei in folgenden Beispielen veranschaulicht.

## Beispiel 1

a) Herstellung des Katalysators

1 455 g $Co(NO_3)_2 \cdot 6H_2O$ werden in 1 000 g destilliertem Wasser gelöst. Diese Lösung wird zu 500 g Zeolith 4A gegeben. Im Rotationsverdampfer wird unter Wasserstrahlvakuum bei ca. 95 bis 100 °C Wasserbadtemperatur die wäßrige Phase abgedampft, bis der Katalysator trocken ist. Der Katalysator wird zerrieben und 3 Stunden bei 300 °C im Ofen calciniert. Der Katalysator enthält dann 26,6 Gew.% Co und 7,8 Gew.% Na, eine wäßrige Aufschlämmung des Katalysators hat einen pH-Wert von 9,8 (Katalysator I).

Zur Herstellung von Strangpreßlingen wird das Katalysatorpulver mit Böhmit AlO(OH) vermengt, ohne Peptisierung eine halbe Stunde geknetet und zu 4 mm-Strangpreßlingen verstrangt. Die Strangpreßlinge werden dann nochmals 3 Stunden bei 300 °C calciniert. Der fertige Katalysator (II) hat folgende Zusammensetzung : 18 Gew.% Co, 5,3 Gew.% Na, 23,2 Gew.% Al, Rest Si und O, $NO_3$-Gehalt 0,5 Gew.%.

b) Umsetzung von Cyclohexyldroperoxid

In einen Rührkolben, versehen mit Innenthermometer und Rückflußkühler, werden 2 g des obengenannten Katalysatorpulvers (I) mit 100 g Oxidationsgemisch, erhalten durch Luftoxidation von Cyclohexan bei 145 °C und 12 bar (Zusammensetzung : 1,81 Gew.% Cyclohexan, 2,61 Gew.% Cyclohexylhydroperoxid, Rest Cyclohexan und Nebenprodukte wie Säuren und Ester), bei 80 °C für 30 Minuten gerührt. Die gaschromatographische Analyse des Reaktionsproduktes ergibt folgende Zusammensetzung : Cyclohexan 2,31 Gew.%, Cyclohexanol 3,15 Gew.%, Cyclohexylhydroperoxid 0,38 Gew.%, Rest Cyclohexan und Nebenprodukte wie Säuren und Ester. Bezogen auf den Peroxidumsatz von 74 Gew.% beträgt die Ausbeute an Cyclohexanon und Cyclohexanol 119 Gew.%.

## Vergleichsbeispiel

728 g $Co (NO_3)_2 \cdot 6H_2O$ werden in 500 ml destilliertem Wasser gelöst. Zu dieser Lösung werden 500 ml Aktivkohlestränge (4 mm) gegeben. Die überstehende Lösung wird abdestilliert und der Katalysator bei 100 °C im Trockenschrank getrocknet. Der Tränkvorgang wird 5 ml wiederholt ; dann ist die gesamte Co-Menge aufgetragen. Nach Trocknung bei 100 bis 110 °C werden die Stränge bei 300 °C unter einer $N_2$-Atmosphäre im Muffelofen calciniert. Der fertige Katalysator enthält 32,6 Gew.% Co. 2 g des Katalysaors werden gemahlen und mit 100 g der obengenannten Oxidationslösung für 30 Minuten bei 80 °C gerührt. Der Peroxidumsatz beträgt 94 Gew.%, die Ausbeute an Cyclohexanon und Cyclohexanol nur 87 Gew.%.

## Beispiel 2

In einem Glasrohr (Innendurchmesser 30 mm, Länge 400 mm), versehen mit Doppelmantel und innenliegendem Thermoelement, werden 200 ml (150 g) des Katalysators II eingefüllt. Bei einer Temperatur von 80 °C, die durch die im Mantel zirkulierende Heizflüssigkeit gehalten wird, erfolgt die Anströmung des Katalysators von unten. Der Zulauf wird mit einer Pumpe konstant gehalten. Das Zulaufgemisch stammt aus der Oxidation von Cyclohexan mit Luft und enthält neben Cyclohexan 1,64 Gew.% Cyclohexanon, 2,40 Gew.% Cyclohexanol, 1,47 G...v.% Cyclohexylhydroperoxid, andere Peroxide 0,06 Gew.%, Säuren und Carbonsäureester 0,9 mäq/l. Zu verschiedenen Zeiten wird der Reaktionsaustrag analysiert. Die effektive Verweilzeit, bezogen auf das Lückenvolumen, beträgt 18,5 Minuten.

| Laufzeit (h) | Peroxid-Umsatz (%) [+] | Cyclohexyl-hydroperoxid-Umsatz (%) [++] | Ausbeute (%) bez. auf Peroxidumsatz |
|---|---|---|---|
| 32 | 91 | 100 | 129 |
| 127 | 90 | 100 | 121 |
| 215 | 90 | 100 | 119 |
| 295 | 90 | 100 | 121 |
| 335 | 90 | 100 | 121 |
| 402 | 90 | 100 | 119 |
| 508 | 90 | 99 | 118 |

[+] Umsatz in %, bezogen auf die Summe der Peroxide im Reaktionsgemisch
[++] Umsatz des Cyclohexylhydroperoxids

Nach der obengenannten Laufzeit ist der Katalysator völlig unverändert, ohne Verkrustung oder wesentlichen Aktivitätsverlust.

Vergleichsbeispiel 2

200 ml (1 110 g) des Co-Kohlekatalysators aus Vergleichsbeispiel 1 werden in die obengenannte Versuchsapparatur eingefüllt und mit dem Oxidationsgemisch beaufschlagt.

| Laufzeit (h) | Peroxid-Umsatz (%) | Cyclohexyl-hydroperoxid-Umsatz (%) | Ausbeute (%) bez. auf Peroxidumsatz |
|---|---|---|---|
| 24 | 98 | 100 | 102 |
| 56 | 96 | 100 | 100 |
| 102 | 91 | 98 | 98 |
| 184 | 85 | 97 | 93 |

Beispiel 3

a) Herstellung des Katalysators

Es werden 50 g Zeolith A in 2 l Wasser suspendiert, auf 80 °C erhitzt und der pH-Wert mit Natronlauge auf 10,5 bis 11 eingestellt. Zu dieser Suspension gibt man eine Lösung von 195 g Co(NO$_3$)$_2$ · 6H$_2$O in 300 ml Wasser. Die Zugabe erfolgt im Verlauf von 1 bis 2 Stunden, wobei man durch gleichzeitige Zugabe einer Lösung von 35 g Natriumhydroxid in 300 ml Wasser für die Aufrechterhaltung des vorgenannten pH-Wertes sorgt. Nach beendeter Zugabe wird die Temperatur auf 100 °C erhöht und nach Abkühlen auf 60 bis 70 °C der Niederschlag abfiltriert und mit 1 500 ml Wasser gewaschen, dessen pH-Wert durch Zugabe von Natronlauge auf etwa 8 gebracht worden ist. Anschließend wird der Filterkuchen 18 Stunden bei 120 °C getrocknet und dann 3 Stunden auf 300 °C erhitzt. Der so erhaltene Katalysator enthält 24,5 Gew.% Cobalt, 6,8 Gew.% Natrium sowie 0,5 Gew.% Nitrat.

b) Zersetzung von Cyclohexylhydroperoxid

In einem autoklaven werden 100 g einer Lösung von 2,0 Gew.% Cyclohexylhydroperoxid und 2,0 Gew.% Cyclohexanon in Cyclohexan zusammen mit 2,0 g des oben beschriebenen Katalysators für 30 Minuten unter Rühren auf 80 °C erhitzt. Anschließend wird der Katalysator abfiltriert, mit Cyclohexan gewaschen und wiederholt verwendet. Das jeweils erhaltene Filtrat wird auf Cyclohexanon, Cyclohexanol und Cyclohexylhydroperoxid analysiert. Die Ergebnisse sind nachfolgend zusammengestellt. Nach dem dritten Gebrauch sind im Reaktionsprodukt 0,03 ppm Cobalt nachweisbar.

| Versuch | Peroxidumsatz (%) | Ausbeute (%) bezogen auf Umsatz |
|---------|-------------------|----------------------------------|
| 1 | 68 | 118 |
| 2 | 68 | 117 |
| 3 | 68 | 118 |

**Ansprüche**

1. Verfahren zur Herstellung von Cyclohexanol und Cyclohexanon durch Behandeln von Cyclohexylhydroperoxid mit Trägerkatalysatoren, die 2 bis 30 Gew.% Cobalt in oxidischer Form, berechnet als Cobalt, enthalten, bei einer Temperatur von 30 bis 160 °C, dadurch gekennzeichnet, daß man Zeolithe als Träger verwendet.

2. Verfahren nach Anspruch 1, dadurch gekennzeichnet, daß man A-, X- oder Y-Zeolithe als Träger verwendet.

3. Verfahren nach den Ansprüchen 1 und 2, dadurch gekennzeichnet, daß man Cyclohexylhydroperoxid enthaltende Gemische verwendet, die bei der Oxidation von Cyclohexan mit molekularem Sauerstoff oder solchen enthaltenden Gasen erhalten wurden.

**Claims**

1. A process for the preparation of cyclohexanol and cyclohexanone by treating cyclohexyl hydroperoxide with a supported catalyst containing from 2 to 30 % by weight, calculated as cobalt, of cobalt in oxidic form, at from 30 to 160 °C, wherein a zeolite is used as the carrier.

2. A process as claimed in claim 1, wherein an A, X or Y zeolite is used as the carrier.

3. A process as claimed in claims 1 and 2, wherein the cyclohexyl hydroperoxide-containing mixture used has been obtained in the oxidation of cyclohexane with molecular oxygen or a gas containing the same.

**Revendications**

1. Procédé de préparation du cyclohexanol et de la cyclohexanone par traitement d'hydroperoxyde de cyclohexyle à une température de 30 à 160 °C avec des catalyseurs sur support, contenant entre 2 et 30 % en poids de cobalt, calculé comme cobalt, sous une forme oxydée, caractérisé en ce que le support est une zéolithe.

2. Procédé suivant la revendication 1, caractérisé en ce que le support est une zéolithe A, X ou Y.

3. Procédé suivant les revendications 1 et 2, caractérisé en ce que l'on emploie des mélanges contenant de l'hydroperoxyde de cyclohexyle, qui sont obtenus lors de l'oxydation du cyclohexane par de l'oxygène moléculaire ou des mélanges gazeux contenant de l'oxygène moléculaire.